# EUROPÄISCHE PATENTSCHRIFT

(11) **EP 4 084 747 B1**
(45) Veröffentlichungstag und Bekanntmachung des Hinweises auf die Patenterteilung: **16.09.2026**
(21) Anmeldenummer: 20828960.3
(22) Anmeldetag: 17.11.2020
(51) Int. Cl.: A61F 5/56

(54) **ANTI-SCHNARCH-VORRICHTUNG**
ANTI-SNORING DEVICE
DISPOSITIF ANTI-RONFLEMENT

(30) Priorität: 26.11.2019 DE 102019131970; 11.03.2020 DE 202020101350 U
(43) Veröffentlichungstag der Anmeldung: 09.11.2022
(73) Patentinhaber: Reinhardt, Thomas J., 34130 Kassel (DE)
(72) Erfinder: Reinhardt, Thomas J., 34130 Kassel (DE)
(74) Vertreter: Reinhardt, Thomas Johannes
(86) Internationale Anmeldenummer: PCT/DE2020/100975
(87) Internationale Veröffentlichungsnummer: WO 2021/104567

(56) Entgegenhaltungen:
- EP-A1- 2 882 384
- DE-A1- 102013 100 898
- DE-C2- 10 011 687
- DE-U1- 29 822 336
- US-A1- 2008 199 824

## Beschreibung

### STAND DER TECHNIK

Die vorliegende Erfindung liegt auf dem Gebiet der Medizintechnik und betrifft eine Vorrichtung zum Verhindern oder Lindern von Schnarchen und Apnoeproblemen, soweit sie auf das sogenannte Zungengrundschnarchen zurückzuführen sind.

Schläft eine betroffene Person in Rückenlage und liegt auf dem Hinterkopf, so bewegt sich, wenn die Person entspannt schläft, der gesamte Zungengrundkörper nach unten und verengt den Luftdurchlass im hinteren Rachenraum. Dies führt bekanntermaßen zu Schnarchgeräuschen. Wird der Luftdurchlass ganz versperrt, so führt dies zu **Atemaussetzern.**

Operationen zur Entfernung oder Teilentfernung des Zäpfchens oder anderer Weichteile im Rachenraum, um Platz zu schaffen oder vibrierende Haut zu entfernen, sind teuer, und wie jede Operation mit Risiko verbunden. Sie sind mit lang anhaltenden Schmerzen in der Abheilphase verbunden und haben manchmal auch nur geringen Erfolg, wenn zu wenig Gewebe entfernt wird.

Zahlreiche Vorrichtungen, die das Problem mechanisch durch Einsetzen irgendwelcher Gerätschaften angehen und ohne Operation auskommen, sind bekannt. Jede einzelne Vorrichtung hat jedoch individuell feststellbare Nachteile. Schlafmasken zur Sauerstoffversorgung sind beispielsweise aufwändig und teuer, das Anlegen ist unkomfortabel. Wenn die Person sich im Schlaf viel bewegt, und die Maske verrutscht, kann sie ihre Wirkung ganz oder teilweise verlieren.

Zahnschienen können vom Zahnarzt individuell an die Person angepasst werden. Sie fixieren den Unterkiefer relativ zum Oberkiefer etwas nach vorn verschoben und verbessern dadurch den Durchgang von Luft durch die in Rückenlage beengte Kehle im Rachenraum, was das Schnarchen vermindert. Sie sind jedoch häufig nicht wirksam genug, verändern manchmal die Bissstellung und können zu Muskelschmerzen im Kiefer führen.

Andere Vorrichtungen wie die aus der Deutschen Patentschrift DE 196 36 680 C1, oder die aus dem Gebrauchsmuster DE 298 22 336 U1 können einen unangenehmen Würgereflex auslösen, und weisen Einzelteile auf, die man verschlucken könnte oder die versehentlich in die Luftröhre geraten könnten.

Die Deutsche Offenlegungsschrift DE 10 2008 041 989 A1 offenbart eine Unterkieferbissschiene, an der mittels daran vorgesehener Häkchen ein Band befestigt werden kann. Das Band wiederum kann mit einem piercingähnlichen Implantat verbunden werden, das auf der Oberseite oder auf der Unterseite der Zunge befestigbar ist. Das Implantat besitzt ein knopfartiges Element, das durch einen Schlitz in dem besagten Band geführt werden kann, wodurch eine Verbindung zwischen Zunge und Zähnen hergestellt ist. Nachteilhaft daran ist, dass die Befestigung zwischen Knopf und Schlitz aufgrund der Enge im Mundraum eine sehr mühsame Angelegenheit ist. Dies trifft sogar dann zu, wenn der am Piercing angebrachte Knopf auf der Oberseite der Zunge vorgesehen ist. Wenn auf der Unterseite der Zunge angebracht, ist das Binden und Lösen der Knopf-Schlitzverbindung noch schwieriger. Aus ästhetischen und diversen anderen Gründen möchten es jedoch viele Menschen vermeiden, mit einem Piercing im Mund gesehen zu werden. Beispielsweise erscheint vielen älteren Menschen, die altersbedingt zum Schnarchen neigen, ein sichtbares Piercing nicht als seriös genug, wenn sie in beruflich verantwortungsvoller Position stehen.

Die Deutsche Gebrauchsmusterschrift DE 298 22 336 U1 offenbart ein Teil zur Befestigung am Unterkiefer und soll die Zunge dort mit einem Gummiband festklemmen, das quer über die Zunge verläuft. Hierbei besteht die Gefahr, dass das Gummiband sich löst oder reißt und das Atmen behindert. Es wird bezweifelt, dass das Gummiband die nötige Kraft auf die Zunge ausüben kann, um die Zunge am Zurückfallen zu hindern. Außerdem ist kein technisches Mittel enthalten, um den Unterkiefer relativ zum Oberkiefer vorn zu halten..

Die US-Patentanmeldung US 2008/ 0199824 A1 offenbart eine Unterkiefer-Protrusionsschiene. Ein Nachteil besteht darin, dass sie kein technisches Mittel enthält, um die Zunge relativ zum Oberkiefer vorn zu halten.

Die Deutsche Patentanmeldung DE 10 201 3 100 898 A1 offenbart eine zweiteilige Vorrichtung, die den Unterkiefer durch Anlage zweier Flansche aneinander vorn halten soll und bei der genügend Platz vorhanden sein soll, um die Zunge weit nach vorn strecken zu können. Die Zunge soll dadurch vorn gehalten werden, dass sie "über die unteren Zähne und die vordere Seite des unteren Einsatzes zu der oder auf die Unterlippe nach vorne gedrückt" wird, siehe Ende von Absatz 32. Nachteilig ist bei der Lehre der D3 hierbei, dass die Zunge nicht verlässliche festgehalten werden kann, und dass der Druck auf die Zunge sehr hoch sein kann, wenn sie zwischen die Schneidezähne von Ober- und Unterkiefer zu liegen kommt. Dies kann sogar zu Bissverletzungen an der Zunge führen, wenn der Benutzer unwillkürlich im Schlaf die Zähne zusammenbeißt.

Die Europäische Patentschrift EP 2 882 384 B1 offenbart eine einteilige Vorrichtung gegen Schnarchen und Schlafapnoe. Sie soll den Unterkiefer und die Zunge vorn halten und hat für die Zähne im Backenzahnbereich jeweils Eindellungen, in denen die Zähne Platz haben sollen. Sie wird durch sogenannte "Adams-clasps", also Klammern aus Edelstahl, die sich um den Zahnhals je eines Zahns legen sollen, an dem Oberkiefer befestigt. Die Zunge soll von einem "target", das eine kugelförmige Gestalt besitzen kann und vorn in der Nähe der Schneidezähne sitzt, zum Tasten angelockt werden, wodurch sich eine natürliche Stimulation der Nerven ergeben soll, die die Muskeln der Zunge anregen, sich im Schlaf unwillkürlich vorn zu halten. Bei Menschen mit langer, schmaler Zunge ist die Wirkung des Anlockens möglicherweise nicht ausreichend. Außerdem wird die Zunge gemäß D4 gar nicht festgehalten, sondern nur "angelockt".

Die Aufgabe der vorliegenden Erfindung besteht daher darin, eine Antischnarch- oder Antiapnoe- Vorrichtung zu schaffen, die zumindest bei einem großen Anteil von Patienten nicht vom Zahnarzt an das individuelle Zahnbild angepasst werden muss, und die den Unterkiefer und die Zunge besser vorn halten kann und damit das Schnarchen wirksamer bekämpft.

### VORTEILE DER ERFINDUNG

Die erfindungsgemäßen Gegenstände mit den Merkmalen von Anspruch 1 lösen diese Aufgabe. In den Unteransprüchen finden sich vorteilhafte Weiterbildungen und Verbesserungen des jeweiligen Gegenstandes der Erfindung.

Die Zunge ist naturgemäß schwer zu packen. Die vorliegende Erfindung beruht, wie im Stand der Technik bekannt, auf dem Lösungsansatz, dass die Zunge daran gehindert werden soll, in Rückenlage auf dem Hinterkopf liegend während des Schlafs in den Schlund hinab zu gleiten. Sie enthält dazu den allgemeinen Lösungsgedanken, dass der festhaltende Zugriff an der Zungenoberfläche nur mittels ein oder mehrerer größerer "Haken" oder "Piercings" geschehen kann, oder mit vielen kleinen Haken oder Spitzen, wie sie etwa bei einer rauen Oberfläche oder einer Bürste zu finden sind.

Gemäß einem ersten Aspekt der vorliegenden Erfindung wird eine Vorrichtung zum Lindern von Schnarchen und Apnoeproblemen offenbart, mit einem Verbindungssystem, das die Zunge mit den Zähnen verbindet, so dass ein Hineingleiten des Zungenkörpers in den Schlundbereich vermindert oder vermieden wird, wobei das Verbindungssystem einen festhaltenden Zugriff an der Zungenoberfläche mittels einer rauen, zur Zunge gerichteten Oberfläche bewirkt, insbesondere durch viele kleine Spitzen, das dadurch gekennzeichnet ist, dass das Verbindungssystem ein Aufschiebeelement zum Aufschieben auf mehrere Zähne der linken und rechten Seite des Oberkiefers oder des Unterkiefers enthält, und das Aufschiebeelement in seinen rechts- und linksseitigen Backenzahnbereichen oder in seinen Eckzahnbereichen wenigstens je einen Haken zur Herstellung einer formschlüssigen Verbindung zwischen dem Aufschiebeelement und einer Unterschnittstelle an der Zahnreihe enthält.

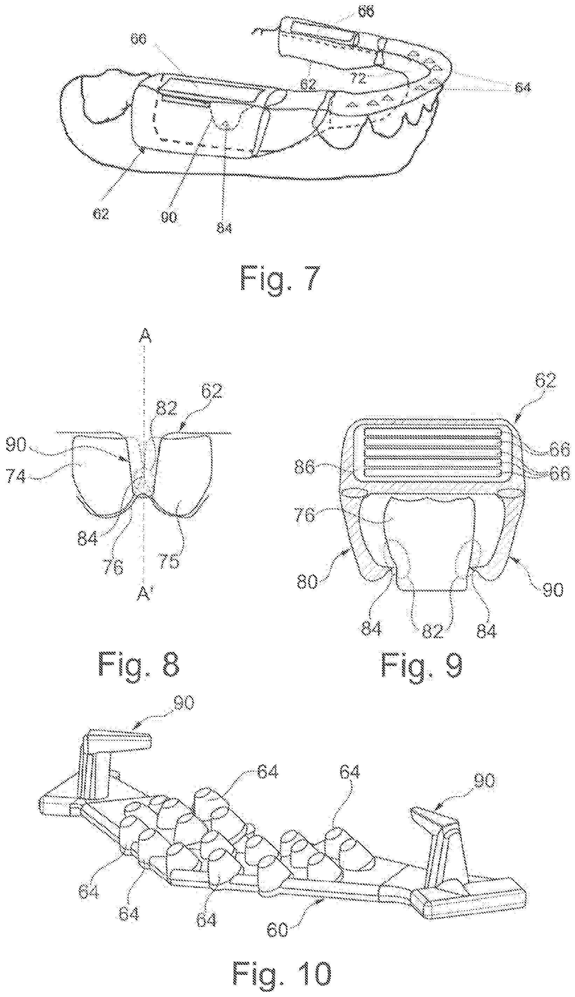

Zur Anwendung wird das Aufschiebeelement am Oberkiefer mit seinen beiden Haken in passenden Interdentalspalten beispielsweise zwischen dem ersten und dem zweiten Backenzahn (zwischen den Zähnen 1.4 und 1.5 bzw. 2.4 und 2.5) verhakt, so dass es einen einigermaßen festen Sitz hat. Dann schiebt der Anwender seine Zunge unter zunehmender Quetschung der Zunge nach vorne, so dass sie zwischen dem Gaumen des Oberkiefers und der rauen Oberfläche des Aufschiebeelements eingefasst wird. Die Spitzen sind bevorzugt als sanft wirkende, rutschhemmende Erhebungen ausgebildet und bilden damit die oben genannte raue Oberfläche. Sie liegen dabei auf der gleichen Seite des Aufschiebeelements wie die Haken, siehe Figur 10. Sie sind vorzugsweise leicht zu den Schneidezähnen hin geneigt, um ein leichtes nach-vorne-Schieben der Zunge zu ermöglichen und ein allzu leichtes Zurückrutschen der Zunge zu verhindern. Diese Ausführungsform gehört nicht zum Gegenstand der gegenwärtig beanspruchten Erfindung. Varianten dessen weisen zusätzlich seitlich angebrachte Magnetpärchen auf, benötigen zur Anwendung zwei solcher Aufschiebeelemente, die auch "Aufbissschienen" oder "Verbindungskörper" genannt werden, um die Zunge zwischen diesen Aufschiebeelementen einzufassen und sind hiernach folgend und anhand der weiteren Figuren beschrieben.

Die vorliegende Erfindung enthält dazu den weiteren allgemeinen Lösungsgedanken, dass der festhaltende Zugriff an der Zungenoberfläche durch einen Verbindungskörper erfolgt, der mit einer gewissen Vielzahl von relativ spitz ausgeformten Elementen ausgestattet ist, die sich an der Zungenoberfläche festsetzen, sich dort etwas eingraben und verhaken, ohne die Haut zu verletzen, wenn diese spitzen Elemente unter einem gewissen Druck auf die Zunge einwirken, wobei der Verbindungskörper auch eine Verbindung zu einem festen Teil des Kiefers oder zu mehreren Kieferpartien rechts / links / oben / unten im Kiefer herstellt.

Als feste Kieferpartie eignen sich hierbei prinzipiell die Zähne, insbesondere die Backenzähne des Oberkiefers und des Unterkiefers. Durch das erfindungsgemäße Verbindungssystem wird ein großer Teil des Zungenkörpers in Richtung der genannten Zähne bzw. des Kinns entgegen der Schwerkraft nach oben gezogen, wenn der Zungenkörper bei einem in Rückenlage entspannt schlafenden Menschen die Tendenz entwickelt, nach unten in den Schlund zu rutschen und dort den Luftdurchlass zu beengen. Durch diesen Zug am Zungenkörper wird der freie Rachenraum, der für den Luftdurchlass notwendig ist, stark vergrößert. Dadurch wird das Schnarchen vermindert oder je nach anatomischen Voraussetzungen ganz vermieden. Apnoezustände werden in vielen Fällen ganz verhindert.

Gemäß dem oben genannten Lösungsansatz der Erfindung wird ein Verbindungskörper zur lösbaren Verbindung der Zunge mit den Zähnen offenbart, der die Zunge in Bezug zu den Zähnen bevorzugt des Oberkiefers festhält und die Bewegung der Zunge hemmt, wenn die Zunge beim entspannten Schlafen in den Schlund zu rutschen droht.

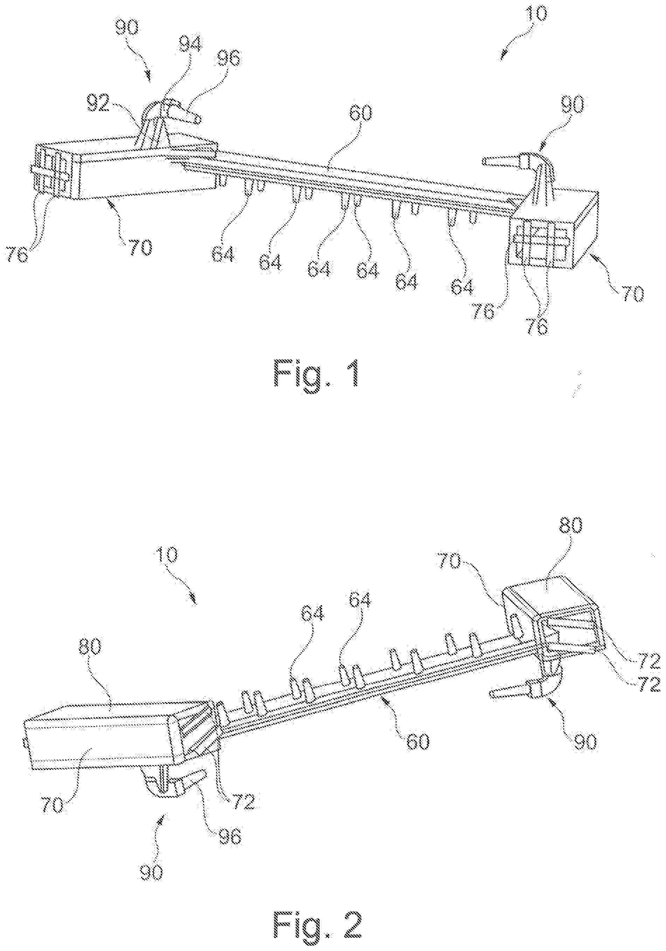

Der Verbindungskörper, zu dem ein Ausführungsbeispiel in den Figuren 1 und 2 abgebildet ist, enthält dazu ein Tragelement für die o.g. relativ spitz ausgeformten Elemente, die nachfolgend auch als rutschhemmende Mittel bezeichnet sind, die beispielsweise in Form von einer gewissen Vielzahl - beispielsweise von 14 - zur Zungenoberfläche gerichteten, leicht abgeflachten Spitzen vorliegen können, die bevorzugt einstückig mit dem Tragelement ausgebildet sein können.

Bevorzugt wirkt ein Tragelement mit den Spitzen auf die Zungenoberseite und ein zweites Tragelement eines zweiten Verbindungskörpers, der baugleich zum ersten Verbindungskörper ausgebildet sein kann, auf die Zungenunterseite. Die Vorrichtung soll zur Anwendung in den Mund genommen werden, wobei die Zunge mit ihrem vorderen, frei beweglichen Bereich zwischen die rutschhemmenden Mittel beider Verbindungskörper zu liegen kommt und dort zwischen diesen eingequetscht wird, wobei sich die rutschhemmenden Mittel ein wenig in das Zungenfleisch eindrücken, ohne jedoch die Zunge zu verletzen. Die Spitzen haben bevorzugt eine solche Form und sind in einer entsprechenden Flächendichte vorhanden, dass Verletzungen oder Schmerzzustände durch "Pieksen" an der Zunge vermieden werden, wenn über die Quetschkraft ein leichter Druck auf die dazwischen liegende Zunge ausgeübt wird.

Die zum Einquetschen der Zungen erforderliche Kraft wird bevorzugt durch magnetische Wirkung von sich anziehenden magnetisch wirksamen Elementen erzielt. Dafür sind an den Tragelementen beider Verbindungskörper bevorzugt seitlich rechts und links der Zunge und außerhalb des Zahnbogens Permanentmagnete in geeigneten Aufnahmen vorgesehen, wobei die Permanentmagnete mit passend vorgesehenen Gegenstücken des anderen Verbindungskörpers zusammenwirken und sich einander anziehen als Magnet-Eisen, oder korrekt orientierte Magnet- Magnet- Pärchen. Sie sind paarweise so an den Verbindungskörpern angeordnet, dass sich die magnetisch anziehenden Teile möglichst großflächig direkt oder getrennt durch eine dünne Gehäusewand eines Gehäuses, in dem sie untergebracht sind, berühren können und einen Haftkontakt herstellen.

Das Gehäuse kann auch nach diversen Seiten mehr oder weniger offen gestaltet sein, soll aber den Magneten bzw. sein Gegenstück durch einen ausreichenden Formschluss fest am Verbindungskörper halten.

Die zum Einquetschen der Zungen erforderliche Kraft kann alternativ auf andere geeignete Weise hergestellt sein, insbesondere durch Schnappverschlüsse oder ineinander einrastende Verbindungselemente, die an den beiden Verbindungskörpern passend zueinander vorhanden sind.

Mindestens einer der Verbindungskörper enthält einen oder mehrere Haken, bevorzugt einen Haken für die rechte Seite und einen Haken für die linke Seite des Oberkiefers, die so ausgebildet sind, dass sie in eine Unterschnittstelle im Spalt zwischen zwei Zähnen derart eindringen können, dass die Haken an den Zähnen die Gegenkraft aufbringen können, die notwendig ist, um die Zunge gegen ihr eigenes Gewicht am Wegrutschen nach unten zu halten.

In einer einfachen Variante stellt nur der Verbindungskörper, der dem Oberkiefer und der Oberseite der Zunge zugeordnet ist, die Verankerung am Kiefer, nämlich an den Oberkieferzähnen zur Verfügung. Das die Zunge an ihrer Unterseite einquetschende Tragelement wird in diesem Fall nur magnetisch an den bereits verankerten Verbindungskörper herangezogen. Der Unterkiefer ist in diesem Fall frei, was zu einem entspannten Schlaf beitragen kann. Diese Ausführungsform gehört nicht zum Gegenstand der gegenwärtig beanspruchten Erfindung.

Bevorzugt hat auch der Verbindungskörper für den Unterkiefer einen Haken zum Einhaken in die rechte und einen Haken zum Einhaken in die linke Zahnreihe des Unterkiefers. Damit kann auch der Unterkiefer in eine protrudierte Stellung relativ zum Oberkiefer gebracht werden und dort auf angenehme Weise gehalten werden, was das Schnarchen weiter reduziert.

Das Tragelement für die rutschhemmenden Mittel kann, wenn es stegförmig und etwa 1 bis 3 mm stark aus dem Kunststoff PA12 hergestellt ist, nach oben oder unten gebogen werden, was beim Einsetzen oder Herausnehmen der Vorrichtung aus dem Mund von Vorteil sein kann, weil sich beim Biegen des Tragelements der Abstand zwischen den Spitzen der Haken verändern lässt.

Die oben genannten magnetisch wirksamen Elemente sind in bevorzugter Weise aus Neodymlegierungen hergestellt und so gebaut und dimensioniert, dass sie im Mund möglichst wenig stören.

Bevorzugt überspannt das Tragelement mit den rutschhemmenden Spitzen den freien Raum zwischen den vorderen Backenzähnen der linken und der rechten Zahnreihe im Oberkiefer bzw. im Unterkiefer. Die Haken sitzen beispielsweise in dem Spalt an der Unterschnittstelle zwischen dem ersten und dem zweiten Backenzahn an der linken bzw. der rechten Zahnreihe des Oberkiefers. Wenn auch das Tragelement für die Unterseite der Zunge in den Unterkiefer eingehakt werden soll, so empfiehlt es sich, für eine leichte Protrusion zu sorgen, indem die Haken zwischen dem zweiten und dem dritten Backenzahn verankert werden. Der Unterkiefer wird bei Kontakt mit den magnetischen Gegenstücken am Tragelement des Oberkiefers dann in einer leichten Protrusionsstellung gehalten. Der besondere Vorteil ist jedoch, dass der Grad der Protrusion für den Anwender stets variierbar ist, was besonders dann von Vorteil ist, wenn der Kiefer wegen der Protrusion wehtut. Der Anwender kann dann bevorzugt stufenweise variiert in eine Normalstellung der Kiefer zueinander zurückkehren, Einzelheiten dazu siehe weiter unten. Aber die Zunge wird dabei immer noch daran gehindert, in den Schlund zu gleiten. Dies ist ein besonderer Vorteil gegenüber Protrusionsschienen nach dem Stand der Technik.

Die Haken lassen sich auch an anderen Zahnzwischenräumen als den genannten verankern, mit oder ohne Protrusion, und wenn es gewollt ist oder anatomisch nicht anders geht, auch in der linken Kieferhälfte auf andere Weise als in der rechten.

Die magnetisch wirksamen Teile sind bevorzugt flach gebaut und quaderförmig. Sie können bevorzugt jeweils in einem passenden Gehäuse untergebracht sein, das seinerseits einstückig mit dem Tragelement ausgebildet sein kann, wenn es durch Spritzguss oder durch 3D-Druck hergestellt werden soll. Die magnetisch wirksamen Teile können aber auch auf die Vorrichtung zum Beispiel mittels eines geeigneten Klebstoffes, wie in Zahnarztpraxen üblicherweise verwendet wird, aufgeklebt sein. Bevorzugt sitzen die Gehäuse bzw. die magnetisch wirksamen Teile nicht auf dem Zahnbogen sondern seitlich und außerhalb des Zahnbogens. Dann hat die Zunge relativ Platz und kann schonend auf großer Fläche eingequetscht werden.

Wenn Permanentmagnet und Gegenstück einen direkten Haftkontakt oder einen Haftkontakt über die zwischen ihnen liegenden Gehäuseflächen ausüben, ist der Unterkiefer relativ zum Oberkiefer sogar fixiert, aber auf eine Weise, die ein leichtes und komfortables Lösen der Fixierung ermöglicht, wie es etwa beim Sprechen, oder wenn die Kiefermuskeln weh tun sollten, erforderlich ist, was eine große Verbesserung zum Stand der Technik darstellt. Je nach Rauigkeit der aneinander anliegenden Haftkontaktflächen und Stärke der magnetischen Anziehungskraft kann der Unterkiefer gegen den Oberkiefer bevorzugt also noch mehr oder weniger leicht verschoben werden. Bevorzugt weisen die Haftkontaktflächen an den Gehäusen der Magnete bzw. der Gegenstücke - oder auch die Magnete und ihre Gegenstücke selbst - ein entsprechendes Muster an jeweils gleich geformten Stegen auf, die von der Form und Anordnung her so gebildet sind, dass sich die Stege der vier Gehäuse (zwei der Vorrichtung am Oberkiefer und zwei der Vorrichtung am Unterkiefer) in mehreren unterschiedlichen Lagen der Gehäuse zueinander ineinander verhaken können.

Die Stege können bevorzugt auch eine gewisse Kurvenform aufweisen, so dass sie immer noch zueinander passen, wenn die Haftkontaktflächen zueinander etwas verdreht werden, was bei den individuellen Rundungsformen der Kieferbögen verschiedener Personen auch erforderlich sein kann.

Um eine große Anziehungskraft zu erreichen, kann der Abstand zwischen Magnet und Gegenstück verringert werden. Dazu können die genannten Stege so lang ausgebildet sein, dass sie von Wand zu Wand verlaufen und das Gehäuse komplett überdecken. Dann kann die unter den Stegen eigentlich verlaufende Decke der Gehäuse, die hierin und in den Figuren auch mit dem Begriff "Haftkontaktfläche 80" bezeichnet ist, auch entfallen. Es bildet sich dann zwischen den Stegen und den Gehäusewänden eine neue Fläche für den Haftkontakt zwischen zwei Gehäusen. Das Gehäuse kann dann auch entsprechend flacher gebaut sein.

Dadurch wirkt das erfindungsgemäße Verbindungssytem doppelt: einmal dadurch, dass es verhindert, dass - bei auf dem Hinterkopf entspannt schlafender Person - der Unterkiefer relativ zum Oberkiefer nach unten rutscht, und weiter dadurch, dass die Zunge relativ zum Oberkiefer in einer weiter oben liegenden Position gehalten wird. Beide Wirkungen vermindern in anerkannter Weise das Schnarchen und addieren sich zu einer verbesserten Gesamtwirkung gegen Schnarchprobleme, ohne dass die Person gravierende Nachteile in Kauf nehmen muss.

Ein weiterer Vorteil besteht darin, dass sich die Zähne nicht mehr berühren, auch wenn die Person während des Schlafs Kaubewegungen macht und zum Zähneknirschen und zu Bruxismus neigt. Daher ist die erfindungsgemäße Vorrichtung prinzipiell auch für an Bruxismus leidende Personen geeignet.

Für eine durchschnittliche Gebissanatomie benötigt die erfindungsgemäße Vorrichtung keinerlei individuelle Anpassung an das persönliche Gebiss, weil sie mit einem spitzen Haken oder einem Pärchen von gegenüberliegenden solchen Haken im rechten Backenzahnbereich und der gleichen Anordnung im linken Backenzahnbereich in eine Unterschnittzone in einem Spalt zwischen zwei Backenzähnen einhaken können. Im Backenzahnbereich finden sich sehr häufig solche Unterschnittzonen. Sehr häufig sind bei Erwachsenen sogar 0,2 mm bis 0,8 mm breite und 0,5 mm bis 3 mm hohe Durchgänge zwischen Zahnfleisch und einer mehr oder weniger geschlossenen Fuge zwischen zwei Zähnen im Backenzahnbereich als brauchbare Unterschnittzone zum Einhaken vorhanden.

Als Material für die erfindungsgemäße Vorrichtung kommt das vielfach für Aufbissschienen verwendete PMMA, oder der etwas weniger spröde, bei Zahnseidesticks üblicherweise verwendete Kunststoff in Frage, wenn die Verbindungskörper durch Spritzguss hergestellt werden sollen. Auch härtere, thermoplastische Elastomere eignen sich. Polyurethan, Polypropylen, ABS sind weitere Kunststoffe, die sich prinzipiell eignen. Für 3-Druck eignet sich der Kunststoff PA12 besonders, da die Spitzen und die Haken, die an sich recht dünn ausgebildet sind, bei Gebrauch nicht so leicht brechen. Je nach Elastizitätsmodul und Bruchfestigkeit des Materials sollte der Haken von seiner Form her so stark geformt sein, dass er beim Einsetzen und beim Herausnehmen der Schiene nicht abbricht. Der Haken kann auch nur gering flexibel gebildet sein, wenn dafür das Tragelement im Ganzen etwas flexibel ist, so dass die beiden Endbereiche des Tragelements mit den Haken beim Einsetzen flexibel etwas auseinandergedrückt werden können, wodurch der Abstand zwischen den Hakenenden vergrößert wird, und die Haken dann beim Loslassen in die Unterschnittzonen hineingleiten. Sollte ein Material nicht biokompatibel genug sein, so kann es dennoch verwendet werden, wenn seine Oberfläche mit einem biokompatiblen Lack beschichtet ist.

Gemäß einem weiteren Lösungsgedanken der Erfindung wird ein Aufschiebeelement zum Aufschieben auf einen oder mehrere Zähne des Unterkiefers und eines zum Aufschieben auf einen oder mehrere Zähne des Oberkiefers zur Herstellung eines Verbindungssystems offenbart, das die Zunge mit den Zähnen verbindet und sie zwischen den Zähnen ohne wegzurutschen festhält, wenn die Zunge zu einem gewissen Teil - bevorzugt mit ihrer Frontpartie - zwischen die beiden Zahnreihen des Ober- und Unterkiefers geschoben wird und zumindest ein leichter Druck von den Zahnreihen über die raue oder mit einigen kleinen Spitzen versehene Oberfläche des Aufschiebeelements auf die obere und untere Zungenoberfläche ausgeübt wird, so dass ein Hineingleiten des Zungenkörpers in den Schlundbereich vermindert oder vermieden werden kann.

Das Aufschiebeelement hat eine raue, oder eine mit einer gewissen Vielzahl von kurzen Spitzen - von beispielsweise eine bis zehn Spitzen pro Quadratzentimetern - oder mit sehr kurzen Borsten versehene, zur Zunge gerichtete Oberfläche, die im Kontakt mit der Zungenoberfläche und unter leichtem Druck auf diese sich mit den vielen kleinen Spitzen oder Borsten entsprechend ihrer Form und ihres Auflagedrucks ein wenig in der Zungenoberfläche verhakt und dadurch eine Rutschhemmung der Zunge bewirkt, wodurch ein Hineingleiten der Zunge in den Schlund während des Schlafes in Rückenlage verhindert wird. Der Anwender verwendet ein Aufschiebeelement bevorzugt für die Unterkieferzahnreihe und gleichzeitig eines für die Oberkieferzahnreihe. Bevorzugt überdeckt ein Aufschiebeelement die Schneidezähne und den Eckzahnbereich, je nach Gegebenheiten auch den vorderen und mittleren Backenzahnbereich. Die für das "Einquetschen" der Zunge wirksame Fläche erstreckt sich entlang der gekrümmten Längsausdehnung des Aufschiebeelements mit einer Breite von bevorzugt 5 mm bis 20 mm.

Die Spitzen haben bevorzugt eine solche Form und sind in einer entsprechenden Flächendichte vorhanden, dass Verletzungen oder Schmerzzustände durch "Pieksen" an der Zunge vermieden werden, wenn über die Kiefer ein leichter Druck auf die dazwischen liegende Zunge ausgeübt wird.

Gemäß einer besonders bevorzugten Ausführungsform ist an der einen Aufbissschiene ein Permanentmagnet und an der anderen Aufbissschiene ein magnetisch anziehbares Gegenstück so angeordnet, dass sie sich genau gegenüber befinden, wenn die Aufbissschienen passgerecht auf ihre jeweilige Zahnreihe aufgeschoben worden sind. Somit ziehen sich die Aufbissschienen etwas an, was den Druck auf die Zunge weiter unterstützt, auch wenn die Kiefermuskulatur entspannt ist. Wenn Permanentmagnet und Gegenstück einen Haftkontakt ausüben, ist der Unterkiefer relativ zum Oberkiefer sogar fixiert, aber auf eine Weise, die ein leichtes und komfortables Lösen der Fixierung ermöglicht, wie es etwa beim Sprechen erforderlich ist, was eine echte Verbesserung zum Stand der Technik darstellt. Dadurch wirkt dieses Verbindungssytem doppelt: einmal dadurch, dass es verhindert, dass - bei auf dem Hinterkopf entspannt schlafender Person - der Unterkiefer relativ zum Oberkiefer nach unten rutscht, und weiter dadurch, dass die Zunge relativ zum Oberkiefer in einer weiter oben liegenden Position gehalten wird. Beide Wirkungen vermindern in anerkannter Weise das Schnarchen und addieren sich zu einer verbesserten Gesamtwirkung gegen Schnarchprobleme, ohne dass die Person gravierende Nachteile in Kauf nehmen muss.

In einer besonderen Variante dessen benötigen die Aufbissschienen keinerlei individuelle Anpassung an das persönliche Gebiss, weil sie mit einem spitzen Haken oder einem Pärchen von gegenüberliegenden solchen Haken im rechten Backenzahnbereich und der gleichen Anordnung im linken Backenzahnbereich in eine Unterschnittzone in einem Spalt zwischen zwei Backenzähnen einhaken können. Im Backenzahnbereich finden sich sehr häufig solche Unterschnittzonen. Sehr häufig sind bei Erwachsenen sogar 0,2 mm bis 0,8 mm breite und 0,5 mm bis 1 mm hohe Durchgänge zwischen Zahnfleisch und einer mehr oder weniger geschlossenen Fuge zwischen zwei Zähnen im Backenzahnbereich als brauchbare Unterschnittzone zum Einhaken vorhanden.

Der Abstand zwischen den Haken bzw. dem Pärchen von Haken und der Innenwange der Aufbissschiene im Schneidezahnbereich ist klein genug, damit die Aufbissschiene im eingehakten Zustand locker in den Zahnbogen hineinpasst ohne an die inneren Flanken der Schneidezähne anzustoßen. Die Innenwange der Aufbissschiene hat nur stabilisierende Wirkung. Sie kann bei geeigneter Materialwahl und Materialstärke der Aufbissschiene auch ganz entfallen, was die universale Anpassungsfähigkeit der Aufbissschiene weiter erhöht und dazu beiträgt, dass die Schiene mit ihren backenzahnseitigen Endstücken leichter nach oben oder unten gebogen werden kann, was beim Einsetzen oder herausnehmen der Schiene aus dem Mund von Vorteil sein kann, wie weiter unten noch näher beschrieben ist.

Die kauflächenseitige Aufbissfläche ist breit genug ausgebildet, damit sie auf jeden Fall noch von den Schneidezähnen erreicht wird und die Zunge bei Bedarf somit zwischen den Schneidezähnen eingeklemmt werden kann. Das Pärchen von Aufbissschienen kann damit in drei Standardgrößen "klein", "mittel" und "groß" hergestellt werden und passt daher nahezu für alle erwachsenen Menschen.

Als Material für die Aufbissschienen kommt das vielfach für Aufbissschienen verwendete PMMA, oder der etwas weniger spröde, bei Zahnseidesticks üblicherweise verwendete Kunststoff in Frage, wenn die Aufbissschienen durch Spritzguss hergestellt werden sollen. Auch härtere, thermoplastische Elastomere eignen sich. Polyurethan, Polypropylen, ABS sind weitere Kunststoffe, die sich eignen. Je nach Elastizitätsmodul und Bruchfestigkeit des Materials sollte der Haken von seiner Form her so stark geformt sein, dass er beim Einsetzen und beim Herausnehmen der Schiene nicht abbricht. Der Haken kann auch gering flexibel gebildet sein, wenn dafür die Aufbissschiene im Ganzen oder sie nur im Schneidezahnbereich etwas flexibel ist, so dass die beiden Backenzahnbereiche beim Einsetzen flexibel etwas auseinandergedrückt werden können und die Haken dann beim Loslassen in die Unterschnittzonen hineingleiten. Der Kunststoff PA2200 eignet sich besonders aufgrund seiner zertifizierten Biokompatibiltät.

Besonders geeignet sind die oben genannten, starken Magnete aus Neodymlegierungen, bevorzugt mit Goldbeschichtung.

### ZEICHNUNGEN

Ausführungsbeispiele der Erfindung sind in den Zeichnungen dargestellt und in der nachfolgenden Beschreibung näher erläutert.
Figur 1 zeigt ein Ausführungsbeispiel perspektivisch von schräg hinten oben, wie es in den Oberkiefer eingesetzt werden kann.
Figur 2 zeigt dasselbe Teil wie in Figur 1 abgebildet, nur von schräg vorn und oben, das in den Unterkiefer eingesetzt werden kann.
Figur 3 zeigt die Vorrichtung aus den Figuren 1 und 2 verankert in einer Unterschnittstelle in einem Zwischenzahnraum der Zahnreihe des Oberkiefers bzw Unterkiefers als schematische Ansichtszeichnung unter Weglassung der sonstigen Zähne, mit zwischengelegter Zunge, und mit einigem, vertikalem Abstand voneinander, so dass die Zunge nicht gequetscht wird.
Figur 4 zeigt die Vorrichtung aus Figur 3 nach Annäherung der Vorrichtungen zueinander mit zwischengelegter Zunge, indem der Mund langsam geschlossen wurde, wobei die Zunge zum besseren Verständnis in einer Querschnittsdarstellung in einer Ebene etwa 1 mm in Richtung der Lippen von der Reihe der Spitzen beabstandet dargestellt ist.
Figur 5 zeigt ein weiteres Ausführungsbeispiel, bei dem die Gehäuse weiter hinten angeordnet sind und die Haftkontaktfläche der Gehäuse mit gekrümmten Stegen versehen ist.
Figur 6 zeigt einen Ausschnitt auf das links in Figur 5 dargestellte Gehäuse von schräg seitlich oben.
Figur 7 zeigt eine Ausführungsform einer Aufbissschiene als Aufschiebeelement für den Unterkiefer, bei der nur der Backenzahnbereich von der Schiene umschlossen ist, der Eckzahn- und Schneidezahnbereich nur eine Stützwange entlang des Zahnbogens auf der Mundinnenseite hat, und sich die Anti-Rutsch Oberfläche an die Stützwange anschließt, und die Außenwange nicht vorhanden ist.
Figur 8 zeigt in einer Seitenansicht von außen auf einen Zahnspalt im Backenzahnbereich eines Unterkiefers einen vor dem Spalt liegenden Haken einer nur zum Teil dargestellten Aufbissschiene,
Figur 9 zeigt in einer freigelegten Schnittdarstellung längs einer Linie A-A' aus Figur 8 auf den Zahnspalt aus Figur 8, aus der Sicht eines Nachbarzahns, mit einem Hakenpärchen einer nur zum Teil dargestellten Aufbissschiene.
Figur 10 zeigt ein weiteres Ausführungsbeispiel, das ohne Permanentmagnete auskommt und bei dem die mit den Spitzen versehene "raue" Oberfläche des Verbindungssystems zwischen Zunge und Zähnen in dieselbe Richtung zeigt, in die sich die Haken erstrecken.
Figur 11 zeigt ein weiteres Ausführungsbeispiel, das zusätzlich zu dem aus Figur 10 Gehäuse für Permanentmagnete aufweist.
Figur 12 zeigt ein weiteres Ausführungsbeispiel, das zusätzlich zu dem aus Figur 11 die mit den Spitzen versehene "raue" Oberfläche auch auf der den Haken entgegengesetzte Seite des Aufschiebeelements trägt aus einer Perspektive von vorne und leicht schräg unten, wenn zur Anwendung im Oberkiefer gedacht.
Figur 13 zeigt das Ausführungsbeispiel aus Figur 12 aus einer Perspektive von hinten und leicht schräg oben.

### BESCHREIBUNG DER AUSFÜHRUNGSBEISPIELE

In den Figuren bezeichnen gleiche Bezugszeichen gleiche oder funktionsgleiche Komponenten.

Figur 1 zeigt ein Ausführungsbeispiel eines erfindungsgemäßen Verbindungskörpers 10 perspektivisch von schräg hinten oben, wie er in den Oberkiefer eingesetzt werden kann.

Figur 2 zeigt denselben Verbindungskörper wie in Figur 1 abgebildet, nur von schräg vorn und oben, der in den Unterkiefer eingesetzt werden kann.

Der Verbindungskörper 10 gemäß Ausführungsbeispiel besteht aus einem flexiblen, harten Kunststoff, im Beispiel aus dem Kunststoff PA12, der für 3D-Druckverfahren geeignet und biokompatibel ist. Er ist mit ausreichend Raum für die Zähne des Oberkiefers und des Unterkiefers ausgebildet, so dass keine individuelle Anpassung an das persönliche Gebiss erforderlich ist. Herstellerseitig sind erfindungsgemäß bereits die genannten Spitzen 64 in das Material eingearbeitet,. Die Spitzen 64 sind in einer angemessenen Anzahl und Flächendichte auf der zur Zunge gerichteten Oberfläche der Schiene vorhanden, im Beispiel 13 Stück auf einem etwa quaderförmigen Tragelement von etwa 4 cm Länge, 8 mm Breite und 1,4 mm Stärke. Die Grundform der Spitzen ist bevorzugt kegelförmig oder pyramidenförmig oder zylinderförmig. Die Höhe im Beispiel beträgt etwa 3 mm. Der Öffnungswinkel an der Spitze beträgt etwa 15°. Die freie Höhe der Spitzen 64 über der Oberfläche des Tragelements sollte je nach Härte des verwendeten Materials und der Anzahl der Spitzen pro Flächeneinheit so angepasst sein, dass die Auflage der Zunge unter geringem Druck keine Schmerzen bereitet, aber die Zunge dennoch am Wegrutschen gehindert wird. Die Spitzen 64 sind daher nicht nadelspitz, sondern abgeflacht, um Schmerz oder Verletzungen zu vermeiden, aber mit relativ kantigem Abschluss, um ein Abgleiten der Zunge wirksam zu verhindern.

Gemäß Ausführungsbeispiel ist nun vorzugsweise an beiden Endabschnitten des Tragelements 60 ein Permanentmagnet in einem quaderförmigen, hohlen Gehäuse 70 mit einer Wandstärke von etwa 0,7 mm vorgesehen. Das Gehäuse 70 sitzt nach Einsetzen der Vorrichtung in den Mund seitlich außerhalb der Zahnreihe in Richtung Wange im Backenzahnbereich. Es ist entsprechend einer durchschnittlichen Form der Außenkontur einer Zahnreihe im menschlichen Gebiss leicht schräg der Außenkontur der Zahnreihe in etwa angepasst angeordnet. Es ist mit dem Tragelement 60 und dem weiter unten beschriebenen Haken 90 zum Verankern der Vorrichtung in der Zahnreihe einstückig ausgebildet.

Der Permanentmagnet bzw. sein magnetisch anziehbares Gegenstück hat die Maße 20 mm lang, 5 mm breit und 1 mm stark, und kann nach 3D- Drucken oder nach dem Gießen der Vorrichtung wie sie in Figur 1 oder 2 abgebildet ist, in das Gehäuse 70 eingelegt werden. Hierzu sind auf der (bei der Anwendung) zur Lippe gerichteten offenen ersten Stirnfläche des Gehäuses zwei Verschlussstege 72 mit den Kanten des Gehäuses verbunden, die etwa parallel zueinander und deutlich schräg relativ zur Kontaktfläche des Gehäuses orientiert sind. Ihr Abstand zueinander ist so groß, dass der Magnet oder sein Gegenstück mit etwas Kraftaufwand hindurchgeschoben werden kann. Die andere offene Stirnfläche des Quaders, die in Rückenlage des Anwenders in Richtung Schlund gerichtet ist, ist durch Quer- und Längsstege 76 gitterartig verschlossen mit einer Gittergröße, die jedenfalls kleiner ist als die einzulegenden Magnete bzw deren Gegenstücke. Daher besteht keine Gefahr, dass sie ihr Gehäuse verlassen und ungewollt in den Schlundbereich gelangen.

Die Gehäuse 70 haben eine Innenhöhe von 2,6 mm, so dass bei Bedarf auch zwei anstelle von nur einem magnetisch wirksamen Quader übereinander angeordnet werden können, um gegebenenfalls die Magnetkraft verstärken zu können.

Zum Gebrauch der Vorrichtung legt man zunächst je einen quaderförmigen Permanentmagneten in die beiden Gehäuse 70 der in Figur 1 dargestellten Vorrichtung für den Oberkiefer jeweils ein. Der Permanentmagnet ist aus einer neodymhaltigen Legierung hergestellt. Er wird zum Einlegen schräg gehalten und durch den Schlitz zwischen den beiden Verschlussstegen hindurchgeschoben.

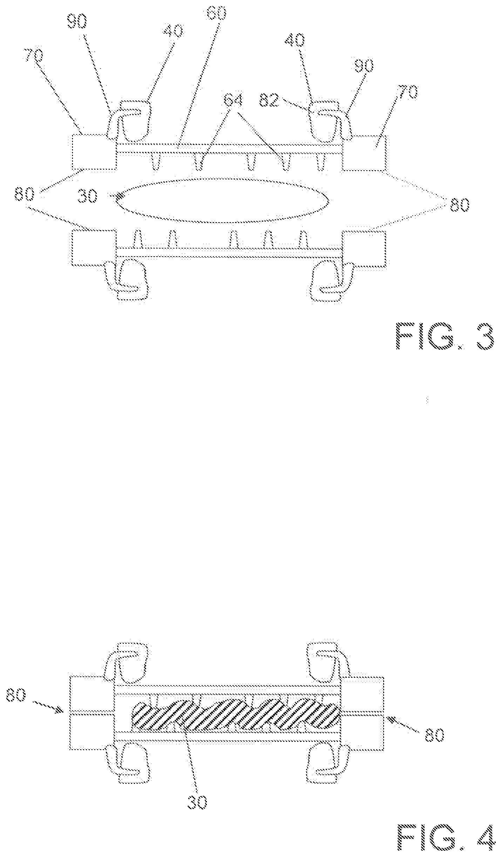

In die Vorrichtung gemäß Figur 2, die identisch mit der aus Figur 1 hergestellt ist, werden zwei magnetisch anziehbare Gegenstücke, zum Beispiel aus ferromagnetischem Eisen eingesetzt, die im Wesentlichen die gleiche Form und Größe haben wie die Permanentmagnete. Alternativ werden die gleichen Permanentmagnete richtig orientiert eingesetzt, so dass die Verbindungskörper sich anziehen, wenn sie in den Mund eingesetzt sind, siehe Figur 3 und 4.

Beim Gebrauch der Vorrichtung sollten sich in bevorzugter Weise die zueinander gerichteten Flächen 80 der vier Gehäuse 70 paarweise berühren, wie es in Figur 4 dargestellt ist. Dabei liegen die Magnete und ihre Gegenstücke mit ihren planen, 5 mm breiten und 20 mm langen Oberflächen an der nur gering größer ausgebildeten Innenoberfläche des jeweiligen Gehäuses an. Die zugehörigen Außenflächen der Gehäuse bilden den Haftkontakt zueinander aus. Sie werden daher hierin "Kontaktflächen" 80 genannt. Die paarweise wirkende magnetische Anziehungskraft ist so groß, dass sie trotz Abschirmung durch die beiderseits vorhandenen, etwa 0,8 mm starken Gehäuseflächen 80 eine so große Anziehungskraft aufeinander ausüben, dass die Zunge, wenn sie zwischen die rutschhemmenden Spitzen eingelegt wird, ausreichend fest dort eingeklemmt wird. Dies haben Versuche gezeigt. Die vorzugsweise durchgängig gleich hoch ausgebildete Höhe der Spitzen 64 relativ zu dem Niveau der Kontaktflächen 80 sollte so eingestellt sein, dass die Spitzen höchstens 1 mm unter diesem Niveau sind. Das passt für die meisten Menschen, wenn die Zunge bei dem stabilen Haftkontakt der Kontaktflächen 80 eingeklemmt sein soll. Wenn die Zunge noch etwas mehr Platz benötigen sollte, kann der Anwender die Spitzen noch vorsichtig geringfügig abfeilen. Ein konstanter Haftkontakt trägt zur Entspannung der Kiefermuskulatur bei.

Im normalen Gebrauch bleiben die Magnete und ihre Gegenstücke in den Gehäusen stecken und fallen nicht durch die zu den Lippen gerichtete Stirnfläche heraus, selbst wenn der Abstand der Verschlussstege 72 aufgrund von hinnehmbaren Toleranzen bei der Herstellung etwas zu groß ausfallen sollte, denn es herrscht immer eine gewisse Anziehungskraft zwischen den magnetisch wirksamen Quadern, wodurch sie sich immer parallel zueinander ausrichten. Aufgrund dieser Parallelität werden sie zusätzlich durch die schräg verlaufenden Verschlussstege 72 gehindert, das Gehäuse zu verlassen. Ein durch grob unsachgemäße Verwendung aus seinem Gehäuse geratener magnetisch wirksamer Quader wird aber dennoch nicht in den Schlund geraten, weil er im Mund der schlafenden Person automatisch zu einem der ihn anziehenden anderen Quader gezogen wird. Die Verschluckgefahr im Schlaf ist daher zu vernachlässigen.

An der der Kontaktfläche 80 gegenüberliegenden Seite der Gehäuse 70 sind jeweils Haken 90 mit ihrem Schaft 92 angebracht. Der Schaft 92 hat eine relativ große Kontaktfläche mit dem Gehäuse und ist bei der Herstellung durch Spritzguss oder 3D-Druck stabil an diesem befestigt. Der Schaft 92 mündet nach konischem Verlauf in ein Krümmungsstück 94, das eine Krümmung vollzieht etwa um 90° nach innen zur Zahnreihe. Das Krümmungsstück 94 geht dann über in eine gerade verlaufende Spitze, die eine Länge von etwa 5 mm aufweist und leicht konischen Verlauf hat, der sich bis zu einer leicht abgerundeten Spitze immer weiter bis auf etwa 0,5 mm verjüngt. Auf diese Weise kann die Spitze bei den meisten Menschen in die üblicherweise vorhandenen Unterschnittstellen im Zwischenzahnbereich zwischen zwei Backenzähnen eingeführt werden. Dadurch wird der Verbindungskörper am Oberkiefer bzw. am Unterkiefer verankert und kann dem Gewicht der bei entspanntem Schlaf nach unten wegrutschenden Zunge eine ausreichend große Gegenkraft entgegen setzen, so dass die Zunge in ihrem zwischen den beiden Tragelementen durch die dort vorhandenen vielen kleinen Spitzen eingequetschten Zustand zuverlässig im vorderen Mundbereich gehalten wird. Der Haken ist bevorzugt einstückig mit dem Tragelement für die rutschhemmenden Mittel durch Spritzguss oder 3D-Druck hergestellt und ist zu einem gewissen Maß biegeelastisch.

In vorteilhafter Weise benötigen die erfindungsgemäßen Verbindungskörper aufgrund ihres Konstruktionsprinzips keine individuelle Anpassung beim Zahnarzt, sondern können zumindest von den meisten erwachsenen Menschen ohne individuelle Anpassung benutzt werden.

Figur 3 zeigt die Vorrichtung aus den Figuren 1 und 2 von vorn in schematischer und vereinfachter Form verankert in einer Unterschnittstelle in einem Zwischenzahnraum der Zahnreihe des Oberkiefers bzw. Unterkiefers als schematische Ansichtszeichnung unter Weglassung der sonstigen Zähne, mit zwischengelegter Zunge 30, und mit einigem, vertikalem Abstand voneinander, so dass die Zunge nicht gequetscht wird.

Figur 4 zeigt die Vorrichtung aus Figur 3 nach Annäherung der Vorrichtungen zueinander mit zwischengelegter Zunge 30, indem der Mund langsam geschlossen wurde bis ein Haftkontakt an den Kontaktflächen 80 entsteht, wobei die Zunge zum besseren Verständnis in einer Querschnittsdarstellung in einer Ebene etwa 1 mm in Richtung der Lippen von der Reihe der Spitzen beabstandet dargestellt ist.

Mit gemeinsamen Bezug zu Figur 3 und 4 ist vereinfacht nur ein Zahn 40 pro Quadrant dargestellt, der die Position der Spitze des Hakens in der Unterschnittstelle zwischen dem Äquator des Backenzahnes und dem Zahnfleisch darstellen soll.

Die Zungenspitze befindet sich weiter vorn in Richtung der Schneidezähne oder kann auch je nach individuellem Gebiss zwischen den Schneidezähnen geschoben werden, um den Zungenkörper als Ganzes weiter vom Schlund zu entfernen. Ein starker Schnarcher kann daher die Zunge möglichst weit nach vorn strecken und sie in der dortigen Lage einquetschen.

Die in Figur 4 schraffiert dargestellte, partiell gequetschte Zunge 30 wird bevorzugt an so vielen Spitzen 64 gehalten, dass der Eingriff der Spitzen keine wesentlichen Schmerzen erzeugt, Dabei empfiehlt es sich, die Spitzen 64 in zwei Reihen so versetzt zueinander anzuordnen, dass sie beim Haftkotakt nicht übereinander angeordnet sind.

Ein positiver Nebeneffekt besteht darin, dass sich die Zähne nicht mehr berühren, auch wenn die Person während des Schlafs Kaubewegungen macht und zum Zähneknirschen und zu Bruxismus neigt. Daher ist die erfindungsgemäße Vorrichtung auch für an Bruxismus leidende Personen geeignet.

Wie aus Figur 4 auch ableitbar ist, reicht es bei einer Person mit nur mäßiger Schnarchneigung prinzipiell aus, wenn nur die Vorrichtung für den Oberkiefer in dessen Zahnreihe mittels Haken rechts und links verankert ist und die Vorrichtung für den Unterkiefer nur die Zunge einklemmt, aber nicht im Unterkiefer verankert ist.

Bevorzugt bildet das Tragelement 60 für die rutschhemmenden Spitzen 64 zusammen mit den beiden Gehäusen 70 und den beiden Haken 90 ein stabiles, einstückig hergestelltes Werkstück.

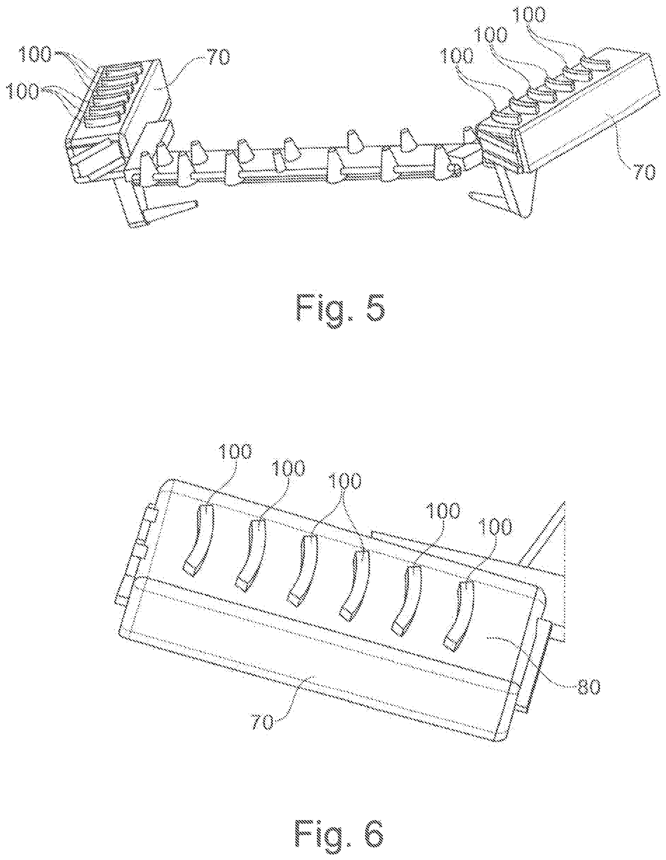

Figur 5 zeigt ein weiteres Ausführungsbeispiel, bei dem die Gehäuse 70 weiter hinten angeordnet sind und die Haftkontaktfläche 80 der Gehäuse 70 mit gekrümmten Stegen 100 versehen ist.

Figur 6 zeigt einen Ausschnitt auf das links in Figur 5 dargestellte Gehäuse 70 von schräg seitlich oben.

Mit gemeinsamen Bezug zu den Figuren 5 und 6 sind die Stege einstückig mit dem Gehäuse 70 durch 3D-Druck gebildet. Die Stege sind Randabschnitte eines ca. 40° Segmentes eines Kreisrandes. Sie haben einen rechteckigen Querschnitt mit einer Breite von etwa 0,7 mm und einer Höhe von etwa 0,8 mm. Sie erstrecken sich im gezeigten Beispiel über etwa zwei Drittel der Breite der Gehäusekontaktfläche 80 und sind immer mit derselben Form in einem Muster aus parallelen Stegen angeordnet, mit einem dazwischen liegenden, lichten Stegzwischenraum von etwa 2,5 mm. Das Muster des links abgebildeten Gehäuses ist etwas versetzt in Relation zum Muster des rechts abgebildeten Gehäuses angeordnet, so dass die Stege 100 zweier solcher, identisch formengleich hergestellter Vorrichtungen aus Figur 5 ineinander zu liegen kommen, wenn die eine im Oberkiefer und die andere im Unterkiefer eingehakt ist und sich die Haftkontaktflächen 80 maximal überlappen. Wenn nun der Anwender die protrudierte Stellung etwas lindern möchte und weniger Protrusion einstellen möchte, so hebt er nur kurz den Unterkiefer aus dem Haftkontakt heraus, so dass die Stege sich beim Zurückschieben des Unterkiefers nicht mehr berühren, schiebt den Unterkiefer in eine etwas weniger protrudierte Stellung und rastet die Stege in der neuen Stellung ein, indem er sich entspannt und damit den magnetisch bewirkten Haftkontakt automatisch wiederherstellt. So kann der Anwender den Grad der Protrusion variieren. Im abgebildeten Beispiel ergeben sich etwa 7 Variationsmöglichkeiten, wobei sich die Gehäuse noch ausreichend überlappen, um genügend Anziehungskraft zwischen den beiden Vorrichtungen im Oberkiefer und Unterkiefer zu erzeugen.

Eine besonders einfache und kostensparende Ausführung entsteht dadurch, dass die beiden Vorrichtungen für Ober- und Unterkiefer exakt gleich mit demselben Formensatz oder denselben 3D-Druckdaten hergestellt sind. Die Haken können dann im Unterkieferbereich, der bei den meisten Menschen einen etwas kleineren Zahnbogen besitzt als der im Oberkiefer beispielsweise zwischen dem 2. und 3. Backenzahn verankert werden und im Oberkiefer zwischen dem ersten und dem zweiten Backenzahn. Hierdurch erreicht man beim Schließen des Mundes meist automatisch eine gewisse Protrusion des Unterkiefers, was das Schnarchen zusätzlich vermindert.

Wenn ein Mensch insgesamt ein etwas kleineres Gebiss als der Durchschnitt hat, kann er die Verbindungskörper meist dennoch benutzen, weil sie bevorzugt pro Kieferquadrant nur einen Haken aufweisen. Um dann einen festen Sitz zu erzielen, kann die Person mit dem kleineren Zahnbogen die Vorrichtungen weiter hinten im Kiefer einhaken.

Ein weiterer großer Vorteil relativ zu den klassischen Protrusionsschienen besteht darin, dass der Verbindungskörper für den Unterkiefer während des Tragens je nach Bedarf und je nach Auftreten von Kieferschmerzen graduell um einige Millimeter gegen den Verbindungskörpers am Oberkiefer verschoben werden kann, ohne dass er gleich komplett aus dem Mund genommen werden muss. Damit ergibt sich eine individuelle Einstellmöglichkeit, die bei vielen anderen klassischen Protrusionsschienen nicht vorhanden ist.

Figur 7 zeigt eine Ausführungsform einer Aufbissschiene als Aufschiebeelement 62 für den Unterkiefer, bei der nur der Backenzahnbereich von der Schiene mit einer Innenwange und einer Außenwange eng umschlossen ist, so dass die magnetischen Zugkräfte auf den Kiefer übertragen werden.

Die magnetisch wirksamen Teile 66 sind mit der Aufbissschiene verbunden, indem sie auf deren Oberfläche mit einem Kleber oder einem geeigneten Zahnzement aufgeklebt sind. In einer Variation sind die magnetisch wirksamen Teile 66 mittels einer formschlüssigen Verbindung mit der Aufbissschiene verbindbar, indem sie durch eine Öffnung in einen Hohlraum mit einem zu den magnetisch wirksamen Teilen passenden Format einschiebbar sind, der parallel zur Bissebene quasi auf der Kaufläche befindlich eine Art "Tasche" - siehe auch die Beschreibung von Figur 9 - bildet. Der Eckzahn- und Schneidezahnbereich weist nur eine Innenwange 72 als Stütz- und Stabilisierungselement entlang des Zahnbogens auf der Mundinnenseite auf, die relativ zu der Innenwange im Backenzahnbereich etwas verkürzt ist und mit ihr einstückig verbunden ist. Die Anti-Rutsch- Oberfläche mit den Spitzen 64 schließt sich einstückig mit ihr an die Innenwange 72 an wie im Beispiel von Figur 8. Die Außenwange im Schneidezahnbereich ist nicht vorhanden, was ein leichteres Tragegefühl bewirkt, der Schiene eine gewisse Biegsamkeit verleiht, die beim Einsetzen der Schiene hilfreich sein kann und ästhetischer aussieht, weil die Lippen nicht nach außen gedrückt werden. Der leichte Druck auf die Zunge kann dennoch angemessen trotz Fehlens der Außenwange ausgeübt werden.

Ein dazu passendes Aufschiebeelement kann analog für den Oberkiefer hergestellt sein. Das Anlegen der Aufbissschienen kann so erfolgen, dass zuerst die Oberkieferschiene und danach die Unterkieferschiene eingesetzt wird, oder umgekehrt. Ebenso können die Schienen wieder entfernt werden.

Somit ergibt sich eine komfortabel tragbare Vorrichtung, die den Unterkiefer daran hindert, sich in Richtung Schlund zu bewegen, wobei die Zunge mit derselben Vorrichtung noch einmal unabhängig davon vom Schlund weg gezogen wird. Um den Benutzerkomfort weiter zu erhöhen, kann die Innenwange im Schneidezahnbereich auch vollständig entfallen.

Ein weiterer großer Vorteil relativ zu den klassischen Protrusionsschienen besteht darin, dass die Unterkieferschiene während des Tragens je nach Bedarf und je nach Auftreten von Kieferschmerzen graduell um einige Millimeter gegen die Oberkieferschiene verschoben werden kann, ohne dass die Schiene gleich komplett aus dem Mund genommen werden muss. Damit ergibt sich eine individuelle Einstellmöglichkeit, die bei vielen anderen klassischen Protrusionsschienen nicht vorhanden ist.

Figur 8 zeigt in einer Seitenansicht von außen auf einen Zahnspalt im Backenzahnbereich eines Unterkiefers einen vor dem Spalt liegenden Haken 90 einer nur zum Teil dargestellten Aufbissschiene 62 gemäß einem weiteren Ausführungsbeispiel der vorliegenden Erfindung.

Figur 9 zeigt in einer freigelegten Schnittdarstellung längs einer Linie A-A' aus Figur 8 auf den Zahn 74 aus Figur 8, aus der Sicht eines Nachbarzahns 75, mit einem Hakenpärchen einer nur zum Teil dargestellten Aufbissschiene 62, wobei beide Haken mit ihren Spitzen 84 in eine Unterschnittstelle 82 an einem Zahnspalt zwischen zwei Backenzähnen einhaken und mit einer in die Aufbissschiene einstückig eingearbeiteten Tasche zur Aufnahme von fünf übereinander angeordneten, plättchenförmigen Permanentmagneten 66.

Mit gemeinsamen Bezug zu Figuren 8 und 9 ist eine Aufbissschiene 62 für den Unterkiefer und eine Aufbissschiene 63 für den Oberkiefer vorgesehen, die jeweils an dem backenzahnseitigen Endabschnitt einen oder mehrere Permanentmagnete 66 oder ein magnetisch anziehbares Gegenstück 70 dazu enthalten, wobei die Aufbissschienen aber so ausgestaltet sind, dass eine individuelle Anpassung an das Gebiss der anwendenden Person in den allermeisten Fällen nicht notwendig ist.

Damit beide Aufbissschienen keine individuelle Anpassung beim Zahnarzt benötigen, um fest auf den Zahnreihen im Backenzahnbereich zu sitzen, sind zwei gegenüberliegende, leicht biegeelastische Haken 90 zur Herstellung einer formschlüssigen Verbindung zwischen der Aufbissschiene und einer Unterschnittstelle 82 an der Zahnreihe vorgesehen. Solche Unterschnittstellen finden sich im Backenzahnbereich der Menschen häufig am Grund der Spalte zwischen zwei Backenzähnen oder am Grund des Spalts zwischen Eckzahn und Backenzahn. Die biegeelastischen Haken 90 besitzen endseitig zwei gegenüberliegende Spitzen 84, die aufeinander zu gerichtet sind. Sie stehen unter einer gewissen Vorspannung, so dass die Spitzen 84 von beiden Seiten in den Zahnspalt eindringen, wenn die Zahnschiene von oben auf die Zahnreihe geschoben wird. Beim korrekt platzierten Aufschieben der Aufbissschiene auf die Zahnreihe der Backenzähne geben die biegeelastischen Haken 90 jeweils etwas nach, wodurch die Spitzen 84 zunächst auseinandergehen. Dadurch lässt sich die Schiene weiter auf die Zahnreihe aufdrücken, wobei die Spitzen 84 der Haken am Rand des Spaltes zwischen zwei Zähnen 74, 75 in Richtung Zahnfleisch 76 gleiten, bis sie in das "Loch" der Unterschnittstelle 82 am Grund des Zahnspaltes quasi einrasten, weil sie ja unter einer angemessen eingestellten Vorspannung stehen. Dadurch bekommt die Aufbissschiene einen ausreichend festen Sitz an ihrer Zahnreihe, um der magnetischen Anziehungskraft der korrespondierenden magnetisch wirksamen Teile 66, 70 Stand zu halten und die Magnetkraft auf Ober- und Unterkiefer zu übertragen.

Die gegenüberliegenden Haken 90 sind einstückig mit der Aufbissschiene durch Formguss oder 3D-Druck herstellbar und sind bevorzugt an der Aufbissschiene so platziert, dass sie in den Zahnspalt zwischen dem zweiten und dritten Backenzahn einrasten können. In einer Variante sind sie so platziert, dass sie zwischen dem ersten und dem zweiten Backenzahn einrasten können.

Die Permanentmagnete 66 und deren Gegenstücke 70 sind mit der jeweiligen Aufbissschiene durch einen biokompatiblen Kleber oder einem zahnmedizinischen Zement verbunden. Alternativ sind sie mit der jeweiligen Aufbissschiene mittels formschlüssiger Verbindung verbindbar anzubringen, indem man sie vor der Anwendung in eine Tasche 86 hineinschiebt, wie oben bei Figur 7 oder unten bei Figur 9 erläutert.

Die universell passenden Aufbissschienen gemäß Ausführungsbeispiel der Figuren 8 und 9 können an den seitlichen Innen- und Außenwangen weniger Material haben, da sie mit weniger Fläche - eben nur der Fläche der Haken 90 - die notwendige Klemmkraft an ihrer Zahnreihe aufbauen. Der Haken 90 mit seiner Spitze 84 ist zum Vergleich mit Ausführungsbeispiel aus Figur 7 in Figur 7 bezüglich der Außenwange im Backenzahnbereich gestrichelt eingezeichnet. Dadurch wird der Vorteil eines angenehmeren Tragekomforts erzielt, denn man hat weniger Fremdkörpermasse im Mund.

In einer bevorzugten Variante des Ausführungsbeispiels der universell passenden Aufbissschiene sind die Haken 90 nur an der Außenwange im Backenzahnbereich vorhanden und die Aufbissschiene ist im Schneidezahnbereich ohne Innenwange oder nur mit einer sehr kurzen Innenwange hergestellt, derart, dass sie etwas flexibel nach oben und unten biegbar ist. Dadurch kann man sie beim Einsetzen mit zwei Händen jeweils zwischen Daumen und Zeigefinger gegriffen etwas aufbiegen, wodurch die Haken 90 links und rechts leicht über die Backenzahnreihe gestreift werden können. Beim Loslassen setzt die Vorspannung der Schiene ein und die Haken 90 verhaken sich in der Unterschnittstelle 82. Zum Lösen der Schiene werden die Schritte in umgekehrter Reihenfolge durchgeführt. Alternativ kann der Anwender auch einen Zahnseidestick zwischen Zahnreihe und Schiene schieben und den Haken 90 aus der Unterschnittstelle heraushebeln.

Bei durchdachter Dimensionierung der universell passenden Aufbissschiene passt auch ein und dieselbe Aufbissschiene einer bestimmten Größe "small", "medium" oder "large" für den Unterkiefer und für den Oberkiefer. Dies hat die Vorteile noch niedrigerer Produktionskosten und erhöhten Komforts bei der Benutzung, weil der Anwender die Schienen nicht verwechseln kann.

Die als "Tasche" bezeichnete Ausnehmung in den diversen Varianten der erfindungsgemäßen Aufbissschiene kann auch einen Schlitz, bevorzugt einen Längsschlitz in Richtung der Einschuböffnung für die magnetisch wirksamen Teile aufweisen, durch den hindurch man ein dünnes Stäbchen, etwa ein Zahnseidestick, stecken kann und die Magnete 66 bzw. deren Gegenstück 70 aus der Tasche wieder hinausschieben kann. Der Schlitz kann auch bewusst verbreitert ausgestaltet sein, um die Magnetkraft möglichst wenig durch das Material der Aufbissschiene abzumildern.

In einem weiteren Ausführungsbeispiel wird eine Protrusionssschiene gemäß der vorangegangenen Beschreibung zu den Figuren 7 bis 9 offenbart, bei der jedoch keinerlei Antirutsch-Oberfläche zum Einklemmen der Zunge vorgesehen ist, sondern nur ein Permanentmagnet mit Gegenstück im Backenzahnbereich rechts und links vorgesehen ist, jedoch sonst keinerlei mechanische "Verzahnung" der beiden Zahnschienen vorgesehen ist. Auch damit lässt sich die oben erwähnte graduelle Verschiebemöglichkeit zur spontanen Schmerzlinderung erreichen.

Figur 10 zeigt ein weiteres Ausführungsbeispiel, bei dem die mit etwas abgerundeten Spitzen 64 versehene "raue" Oberfläche des Verbindungssystems zwischen Zunge und Zähnen in dieselbe Richtung zeigt, in die sich die Haken erstrecken.

Das Verbindungssystem enthält wieder ein Aufschiebeelement, zum Aufschieben auf und zum beiderseitigen Einhaken in die Zahnreihe, das wie oben beschrieben auch als Tragelement 60 für die Spitzen 64 dient. Bei seiner Verwendung im Oberkiefer wie dargestellt, erstreckt es sich von der linken zur rechten Zahnreihe derselben Kieferhälfte, also beispielsweise von der linken Backenzahnreihe quer hinüber zur rechten Backenzahnreihe im Oberkiefer. Es ist dort mit den vorgenannten Haken 90 jeweils in einer Unterschnittstelle rechts und links von außen kommend im jeweiligen Backenzahnbereich verankerbar, wie zuvor beschrieben. Nun sind die sanft ausgebildeten Spitzen 64 , die die Rauigkeit der Oberfläche bestimmen, auf der im Vergleich zu Figur 1 entgegengesetzten flachen Seite des Tragelements 60 angeordnet.

Die sanften Spitzen 64 sind etwas in Richtung Schneidezähne gerichtet, um eine leichte "Widerhakenwirkung" gegenüber der Zungenoberfläche zu erzeugen, um das Wegrutschen der Zunge in Richtung Schlund weiter zu hemmen. Auf diese Weise ist eine besonders vorteilhaft ausgebildete rutschhemmende Fläche gebildet.

Zur Anwendung im Oberkiefer hakt der Benutzer die Vorrichtung rechts und links in passenden Zahnspalten im Backenzahnbereich des Oberkiefers mit den Haken 90 ein. Hat er einen schmalen Kiefer hakt er sie Vorrichtung weiter hinten ein, denn dort ist der Zahnbogen breiter. Hat er einen sehr breiten Kiefer, hakt er sie entsprechend weiter vorn ein. Unsymmetrisches Einhaken funktioniert auch. Die rutschhemmende Fläche verspannt sich damit von links nach rechts im Backenzahnbereich des Oberkiefers und ist nach oben gerichtet. Dann schiebt der Anwender seine Zunge in den engen, freien Raum zwischen Oberkiefergaumen und rutschhemmender Fläche. Die Unterseite der Zunge kommt dabei in Kontakt mit den Spitzen 64 auf der rutschhemmenden Fläche. Da die Zunge normalerweise an ihrer Spitze dünner ist und nach hinten dicker wird, liegt die Zunge beim Schieben nach vorn immer enger zwischen Gaumenfläche und rutschhemmender Fläche an je weiter sie nach vorn geschoben wird. Der Gaumen und die rutschhemmende Fläche üben dabei einen zunehmenden Druck auf die Zunge aus. Die Haken 90 bilden dafür das Gegenlager in ihrer jeweiligen Unterschnittstelle der Zahnreihe liegend.

Irgendwann beim Nach-vorn-Schieben ist ein "Festhaltepunkt" erreicht, an dem die Zunge aufgrund des dann ausreichenden Drucks der rutschhemmenden Oberfläche des Tragelements 60 nicht mehr von alleine zurückrutscht, wenn sich der Anwender entspannt und auf dem Rücken liegt, wie es im Schlaf der Fall ist. Da die Querschnittsfläche der Zunge und ihr Verlauf von der Zungenspitze nach hinten bei jedem Menschen individuell verschieden ist, und dies auch für die Wölbung des Oberkiefergaumens zutrifft, ist dieser Festhaltepunkt ebenso individuell.

Aus diesem Grund ist das Tragelement 60 nicht allzu tief ausgebildet, so dass ein freier Raum zwischen dessen Vorderkante und den Scheidezähnen vorhanden ist, durch den hindurch sich die Zungenspitze schieben kann, wenn es notwendig ist, etwa bei Anwendern mit einer schmalen und dünnen, eher langgestreckten Zunge. Die Zunge kann dann immer weiter nach vorn geschoben werden und rollt sich gegebenenfalls an den Innenflanken der Schneidezähne wieder nach unten ein. Auf diese Weise kann auch eine lange dünne Zunge weit hinten festgehalten werden und macht den Schlund frei für das schnarchfreie Atmen.

In dieser Basisversion des erfindungsgemäßen Verbindungssystems benötigt das Aufschiebeelement keine Permanentmagneten an den Seiten und auch kein zweites Aufschiebeelement, wenn der Anwender in Kauf nimmt, dass sein Unterkiefer beim Schlafen in Rückenlage etwas zurückfallen wird.

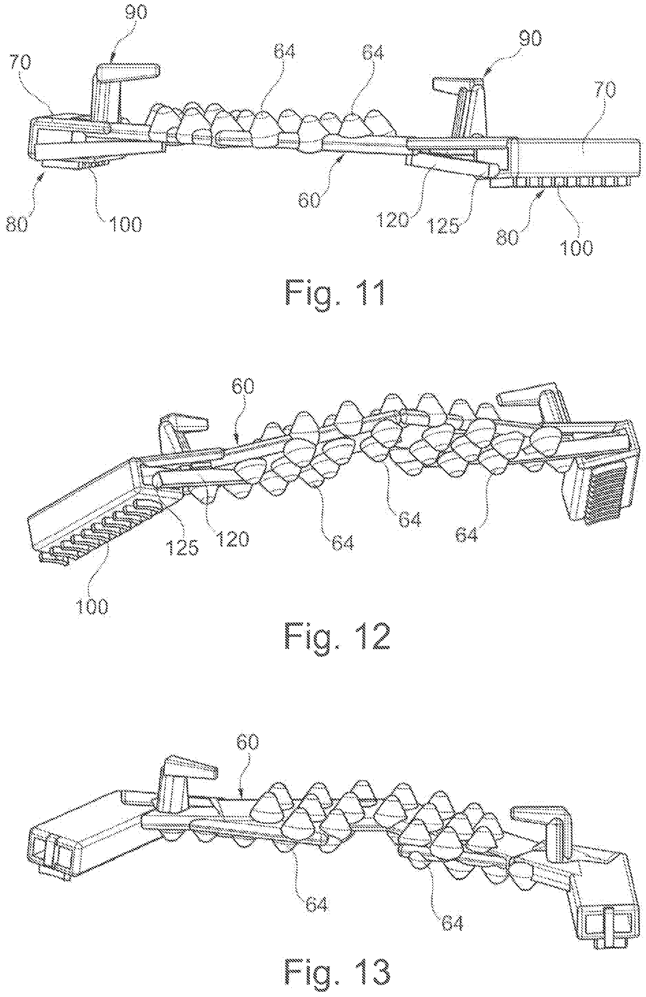

Figur 11 zeigt ein weiteres Ausführungsbeispiel, das zusätzlich zu dem aus Figur 10 Gehäuse 70 für Permanentmagnete aufweist, um durch die magnetische Anziehungskraft und die Haftung an den Kontaktflächen 80 den Unterkiefer zu fixieren, so dass er in Rückenlage nicht nach unten gleitet. Dies wird auch durch die Verhakung der Rundstege 100 ineinander unterstützt, wie im Zusammenhang mit Figur 6 beschrieben. Vor den zu den Schneidezähnen gerichteten Öffnungen der Gehäuse 70 sind am Tragelement 60 Verschlussstege 120 befestigt, an ihrem anderen Ende 125 frei in der Luft sind und daher gegen ihre natürlich vorhandene Vorspannung elastisch von der Öffnung weg gebogen werden können, um die Permanentmagnete oder ihre ferritischen oder permanentmagnetischen Gegenstücke in die Gehäuse 70 einschieben zu können. Wenn man die Verschlussstege 120 löslässt, nehmen sie ihre ursprüngliche Lage wieder ein, wie dargestellt und blockieren die Öffnung, so dass die Magnete sicher im Gehäuse verbleiben, auch wenn das Aufschiebeelement geschüttelt wird.

Figur 12 zeigt ein weiteres Ausführungsbeispiel, das zusätzlich zu dem aus Figur 11 die mit den Spitzen 64 versehene "raue" Oberfläche auch auf der den Haken entgegengesetzte Seite des Aufschiebeelements, siehe auch die Beschreibung zu den Figuren 1 bis 5, trägt, aus einer Perspektive von vorne und leicht schräg unten, wenn zur Anwendung im Oberkiefer gedacht.

Figur 13 zeigt das Ausführungsbeispiel aus Figur 12 aus einer Perspektive von hinten und leicht schräg oben.

Gemäß Figuren 12 und 13 ist die erfindungsgemäße Vorrichtung gemäß der zuletzt genannten Ausführung mit einem Tragelement 60 versehen, das auf beiden Seiten, also auf dessen Unterseite und Oberseite die rutschhemmende Oberfläche mit den sanften Spitzen 64 aufweist. Dies hat den Vorteil, dass der Anwender die Wahl hat, seine Zunge zwischen die rutschhemmenden Flächen zweier solcher Vorrichtungen oder, wie bei Figur 11 beschrieben, zwischen Gaumen und rutschhemmender Fläche zu legen, um sie von dem erfindungsgemäßen Verbindungssystem festhalten zu lassen.

Obwohl die vorliegende Erfindung anhand von bevorzugten Ausführungsbeispiels vorstehend beschrieben wurde, ist sie darauf nicht beschränkt, sondern auf vielfältige Weise modifizierbar.

Die nachfolgend beschriebene Ausführungsform stellt eine Ausführungsform gemäß der Erfindung dar für Anwender, bei denen das Zahnbild nicht dem Durchschnitt entspricht, weil Zähne fehlen oder die genannten Unterschnittstellen nicht vorhanden oder aus anderen Gründen nicht brauchbar sind, erfolgt die Verbindung zum Kiefer nicht durch einen Haken, sondern dadurch, dass ein individuell angepasstes Zahnschienen-Passstück mit in den Verbindungskörper integriert ist. Das Passstück wird bevorzugt von einem Zahnarzt aus einem haftenden Material mittels Zahnabdruck derart hergestellt, dass es eng an die Zähne des Zahnbereichs angearbeitet ist und knapp über den Äquator der Zähne im betroffenen Zahnbereich hinausgeht. Somit ist eine ausreichende Gegenkraft zum Halten der Zunge durch die dann vorhandene Haftkraft zwischen Passstück und Zahnbereich hergestellt. Das Passstück muss sich nicht notwendig über den gesamten Zahnbogen erstrecken, sondern kann beispielsweise nur einen oder zwei oder drei Zähne umfassen. Es können auch Verbindungskörper hergestellt werden, die je nach Bedarf zur Erzeugung der Gegenkraft eine Kombination aus Haken und haftenden Passstücken aufweisen. Das Passstück kann dann mittels eines geeigneten Klebstoffes auf einen serienmäßigen Verbindungskörper aufgeklebt werden, und der Haken kann bleiben oder vorher vom Serienkörper abgetrennt werden.

Wenn Schnappverschlüsse oder ineinander einrastende Verbindungselemente anstelle der magnetisch wirksamen Verbindungselemente verwendet werden, sollten die Schnappverschlüsse bzw. die Rastungen so gearbeitet sein, dass sie durch die Kraft der Kiefermuskulatur problemlos wieder zu öffnen sind.

## Patentansprüche

1. Vorrichtung zum Lindern von Schnarchen und Apnoeproblemen, mit einem Verbindungssystem (10), das die Zunge mit den Zähnen verbindet, so dass ein Hineingleiten des Zungenkörpers in den Schlundbereich vermindert oder vermieden wird, wobei das Verbindungssystem eine Aufbissschiene (62) enthält, die so geformt ist, dass sie zum Aufschieben auf einen oder mehrere Unterkieferzähne geeignet ist, und eine weitere Aufbissschiene (63) enthält, die so geformt ist, dass sie zum Aufschieben auf einen oder mehrere Oberkieferzähne geeignet ist,
wobei die Aufbissschienen (62, 63) jeweils ein Tragelement (60) mit einer zur Zunge gerichteten Oberfläche besitzen, die rutschhemmende Mittel (64) aufweist, um das Wegrutschen der Zunge in Richtung Schlund zu hemmen,
**dadurch gekennzeichnet, dass** die Tragelemente (60) den freien Raum zwischen den vorderen Backenzähnen der linken und der rechten Zahnreihe im Oberkiefer bzw. im Unterkiefer überspannen,
a) wobei die Aufbissschienen (62, 63) jeweils in ihren Backenzahnbereichen oder in ihren Eckzahnbereichen ineinander einrastende Verbindungselemente aufweisen, und
b) die Aufbissschienen (62, 63) ein integriertes Passstück enthalten, das dazu eingerichtet ist, eng an die Zähne angearbeitet zu sein und knapp über den Äquator der Zähne im betroffenen Zahnbereich hinauszugehen.

2. Vorrichtung zum Lindern von Schnarchen und Apnoeproblemen, mit einem Verbindungssystem (10), das die Zunge mit den Zähnen verbindet, so dass ein Hineingleiten des Zungenkörpers in den Schlundbereich vermindert oder vermieden wird, wobei das Verbindungssystem eine Aufbissschiene (62) enthält, die so geformt ist, dass sie zum Aufschieben auf einen oder mehrere Unterkieferzähne geeignet ist, und eine weitere Aufbissschiene (63) enthält, die so geformt ist, dass sie zum Aufschieben auf einen oder mehrere Oberkieferzähne geeignet ist,
wobei die Aufbissschienen (62, 63) jeweils ein Tragelement (60) mit einer zur Zunge gerichteten Oberfläche besitzen, die rutschhemmende Mittel (64) aufweist, um das Wegrutschen der Zunge in Richtung Schlund zu hemmen,
**dadurch gekennzeichnet, dass** die Tragelemente (60) den freien Raum zwischen den vorderen Backenzähnen der linken und der rechten Zahnreihe im Oberkiefer bzw. im Unterkiefer überspannen,
a) wobei die Aufbissschienen (62, 63) jeweils in ihren Backenzahnbereichen oder in ihren Eckzahnbereichen Permanentmagnete (66) enthalten, die so angeordnet sind, dass sie mit passend vorgesehenen Gegenstücken der anderen Aufbissschiene zusammenwirken und sich einander anziehen, und
b) die Aufbissschienen (62, 63) ein integriertes Passstück enthalten, das dazu eingerichtet ist, eng an die Zähne angearbeitet zu sein und knapp über den Äquator der Zähne im betroffenen Zahnbereich hinauszugehen.

3. Vorrichtung nach Anspruch 2, wobei an den Aufbissschienen (62, 63) Gehäuse (70) zur Aufnahme der Permanentmagnete (66) und deren Gegenstücke vorgesehen sind,
wobei die Gehäuse (70) jeweils eine Gehäusekontaktfläche (80) zur Herstellung eines magnetischen Haftkontakts mit einem jeweils anderen Gehäuse (70) der anderen Aufbissschiene aufweisen,
und die Gehäusekontaktflächen (80) ein Muster aus Stegen (100) aufweisen, die bei Haftkontakt ineinander zu liegen kommen,
wobei die Muster so gebildet sind, dass sich die Stege (100) in mehreren unterschiedlichen Lagen der Gehäuse zueinander ineinander verhaken können.

4. Vorrichtung nach einem der vorstehenden Ansprüche, wobei sie mittels Gussverfahren oder 3D-Druck hergestellt ist.

5. Vorrichtung nach dem vorstehenden Anspruch, wobei die Vorrichtungen für Ober- und Unterkiefer exakt gleich mit demselben Formensatz bzw. denselben 3D-Druckdaten hergestellt sind.

## Claims

1. A device for alleviating snoring and apnea problems, comprising a connection system (10) that connects the tongue to the teeth, thus reducing or preventing the tongue body from slipping into the pharynx region, wherein the connection system includes a bite splint (62) shaped to be suitable for sliding onto one or more lower teeth, and a further bite splint (63) shaped to be suitable for sliding onto one or more upper teeth,
wherein the bite splints (62, 63) each have a support element (60) with a surface facing the tongue, which has anti-slip means (64) to prevent the tongue from sliding towards the pharynx,
**characterized in that** the support elements (60) span the free space between the anterior molars of the left and right dental arches in the upper jaw and the lower jaw, respectively,
a) wherein the bite splints (62, 63) each have interlocking connecting elements in their molar regions or in their canine regions, and
b) the bite splints (62, 63) comprise an integrated fitting piece designed to fit closely to the teeth and to extend just beyond the equator of the teeth in the affected dental region.

2. A device for alleviating snoring and apnea problems, comprising a connection system (10) that connects the tongue to the teeth, thus reducing or preventing the tongue body from slipping into the pharynx region, wherein the connection system includes a bite splint (62) shaped to be suitable for sliding onto one or more lower teeth, and a further bite splint (63) shaped to be suitable for sliding onto one or more upper teeth,
wherein the bite splints (62, 63) each have a support element (60) with a surface facing the tongue, which has anti-slip means (64) to prevent the tongue from sliding towards the pharynx,
**characterized in that** the support elements (60) span the free space between the anterior molars of the left and right dental arches in the upper jaw and the lower jaw, respectively,
a) wherein the bite splints (62, 63) each contain permanent magnets (66) in their molar regions or in their canine regions, which are arranged to interact with appropriately provided counterparts of the other bite splint and attract each other, and
b) the bite splints (62, 63) comprise an integrated fitting piece designed to fit closely to the teeth and to extend just beyond the equator of the teeth in the affected dental region.

3. Device according to claim 2, wherein housings (70) for receiving the permanent magnets (66) and their counterparts are provided on the bite splints (62, 63),
wherein the housings (70) each have a housing contact surface (80) for producing a magnetic occlusal contact with a respective other housing (70) of the other bite splint, and the housing contact surfaces (80) have a pattern of webs (100) arranged into one another when in occlusal contact,
wherein the patterns are formed such that the webs (100) can interlock in several different positions of the housings relative to each other.

4. Device according to one of the preceding claims, wherein it is manufactured by means of a casting process or 3D printing.

5. Device according to the preceding claim, wherein the devices for the upper and lower jaw are manufactured exactly the same using the same set of molds or the same 3D printing data.

## Revendications

1. Dispositif pour atténuer les problèmes de ronflement et d'apnée, comprenant un système de liaison (10) qui relie la langue aux dents de manière à réduire ou à empêcher le glissement du corps de la langue vers la région pharyngée, dans lequel le système de liaison comprend une gouttière occlusale (62) conformée pour pouvoir être glissée sur une ou plusieurs dents mandibulaires, et comprend une autre gouttière occlusale (63) conformée pour pouvoir être glissée sur une ou plusieurs dents maxillaires,
dans lequel les gouttières occlusales (62, 63) comportent chacune un élément de support (60) présentant une surface orientée vers la langue qui est pourvue de moyens antidérapants (64) destinés à empêcher la langue de glisser vers le pharynx,
**caractérisé en ce que** les éléments de support (60) s'étendent sur l'espace libre situé entre les molaires antérieures des rangées dentaires gauche et droite, respectivement au niveau de la mâchoire supérieure et de la mâchoire inférieure,
a) dans lequel les gouttières occlusales (62, 63) comportent chacune des éléments de liaison à emboîtement situés dans leurs régions molaires ou dans leurs régions canines, et
b) les gouttières occlusales (62, 63) contiennent une partie d'ajustement intégrée configurée pour s'adapter étroitement aux dents et pour s'étendre juste au-delà de l'équateur des dents dans la région dentaire concernée.

2. Dispositif pour atténuer les problèmes de ronflement et d'apnée, comprenant un système de liaison (10) qui relie la langue aux dents de manière à réduire ou à empêcher le glissement du corps de la langue vers la région pharyngée, dans lequel le système de liaison comprend une gouttière occlusale (62) conformée pour pouvoir être glissée sur une ou plusieurs dents mandibulaires, et comprend une autre gouttière occlusale (63) conformée pour pouvoir être glissée sur une ou plusieurs dents maxillaires,
dans lequel les gouttières occlusales (62, 63) comportent chacune un élément de support (60) présentant une surface orientée vers la langue qui est pourvue de moyens antidérapants (64) destinés à empêcher la langue de glisser vers le pharynx,
**caractérisé en ce que** les éléments de support (60) s'étendent sur l'espace libre situé entre les molaires antérieures des rangées dentaires gauche et droite, respectivement au niveau de la mâchoire supérieure et de la mâchoire inférieure,
a) dans lequel les gouttières dentaires (62, 63) contiennent chacune des aimants permanents (66) dans leurs régions molaires ou canines, disposés pour interagir avec des éléments complémentaires prévus à cet effet sur l'autre gouttière dentaire et pour s'attirer mutuellement, et
b) les gouttières occlusales (62, 63) contiennent une partie d'ajustement intégrée configurée pour s'adapter étroitement aux dents et pour s'étendre juste au-delà de l'équateur des dents dans la région dentaire concernée.

3. Dispositif selon la revendication 2, dans lequel des logements (70) destinés à recevoir les aimants permanents (66) et leurs homologues sont prévus sur les gouttières occlusales (62, 63),
dans lequel les logements (70) présentent chacun une surface de contact de logement (80) pour établir un contact d'adhérence magnétique avec un logement (70) correspondant situé sur l'autre gouttière occlusale,
et les surfaces de contact de logement (80) présentent un motif de nervures (100) qui s'engrènent lors du contact d'adhérence,
dans lequel les motifs sont formés de telle sorte que les nervures (100) peuvent s'imbriquer les unes dans les autres dans une pluralité de positions relatives différentes des logements.

4. Dispositif selon l'une quelconque des revendications précédentes, fabriqué au moyen d'un procédé de moulage ou par impression 3D.

5. Dispositif selon la revendication précédente, dans lequel les dispositifs destinés aux mâchoires supérieure et inférieure sont fabriqués de manière à être exactement identiques, en utilisant le même jeu de moules ou les mêmes données d'impression 3D.
